(19) Europäisches Patentamt
European Patent Office
Office européen des brevets

(11) Veröffentlichungsnummer: **0 188 285**
**A2**

(12) # EUROPÄISCHE PATENTANMELDUNG

(21) Anmeldenummer: **86100484.4**

(22) Anmeldetag: **16.01.86**

(51) Int. Cl.⁴: **C 08 G 77/16**
C 08 L 83/06, C 08 K 9/06
C 08 J 3/24, A 61 K 6/10

(30) Priorität: **17.01.85 DE 3501412**

(43) Veröffentlichungstag der Anmeldung:
**23.07.86 Patentblatt 86/30**

(84) Benannte Vertragsstaaten:
**CH DE FR GB IT LI NL**

(71) Anmelder: **WACKER-CHEMIE GMBH**
**Prinzregentenstrasse 22**
**D-8000 München 22(DE)**

(72) Erfinder: **Hefner, Heinz, Dr. Dipl.-Chem.**
**Vivaldistrasse 2**
**D-8263 Burghausen(DE)**

(72) Erfinder: **Garhammer, Arnold**
**Pfarrkirchner Strasse 56**
**D-8346 Simbach(DE)**

(72) Erfinder: **Eckhart, Louis, Dr. Dipl.-Chem.**
**Wacker Chemicals East Asia New Hoechst Building**
**8/10/16 Akasaka Minato/ku Tokyo 107(JP)**

(54) **Verfahren zum Herstellen von Organopolysiloxanelastomeren und eine Anwendung dieses Verfahrens.**

(57) Bei Gemischen, die durch Vermischen von in den endständigen Einheiten je eine Si-gebundene Hydroxylgruppe aufweisendem Diorganopolysiloxan mit einer durchschnittlichen Viskosität von 500 bis 6000 mPa.s bei 25°C mit anorganischem Oxyd mit einer Oberfläche von mindestens 50 $m^2/g$ in einer Menge von 2 bis 35 Gewichtsprozent anorganischem Oxyd, bezogen auf das Gesamtgewicht von Diorganopolysiloxan und anorganischem Oxyd sowie mit Wasser und mit das anorganische Oxyd hydrophobierender Organosiliciumverbindung und Entfernung der niedriger als Diorganopolysiloxan siedenden Bestandteile aus dem Gemisch und gegebenenfalls erfolgendem weiteren Zusatz von Wasser hergestellt wurden, ist bei ihrer Lagerung vor der Vernetzung keine Absetzung von Füllstoff zu beobachten. Derartige Gemische werden nach Vermischen mit Vernetzungsmittel und Kondensationskatalysator auf abzuformenden Oberflächen, insbesondere Zähnen und/oder Schleimhaut, vernetzen gelassen. Dieses Verfahren eignet sich ausgezeichnet zur Herstellung von Dentalkorrektur-Abdrücken bzw. als Teil der Herstellung von Dentalkorrektur-Abdrücken.

EP 0 188 285 A2

Wacker-Chemie GmbH

München, den 26.07.1984
PAT/Dr.Ru/hu

Wa 8336-S

Verfahren zum Herstellen von Organopolysiloxanelastomeren und eine Anwendung dieses Verfahrens.

---

Aus US 4 389 496, ausgegeben 21. Juni 1983, B. Leusner et al., Bayer Aktiengesellschaft, sind bereits Pasten zur Herstellung genauer Abformungen von Zähnen und Schleimhaut bekannt. Diese Pasten werden aus in den endständigen Einheiten je eine Si-gebundene Hydroxylgruppe aufweisendem Diorganopolysiloxan mit verhältnismäßig geringer Viskosität, einem organischen Thixotropiermittel und Calciumsilikat bereitet.

Es ist Aufgabe der Erfindung, ein Verfahren zum Herstellen von Organopolysiloxanelastomeren zu schaffen, wobei nach Lagerung der dabei verwendeten Massen vor deren Verwendung zur Erzeugung der Elastomeren keine Absetzung von Füllstoff zu beobachten ist, die Massen eine genügend niedrige Viskosität haben, um die feinsten Einzelheiten der Oberflächen, von denen Abdrücke gemacht werden sollen, genau wiederzugeben, und trotzdem, weil sie standfest sind, von den Oberflächen, von denen Abdrücke gemacht werden sollen, vor ihrer Vernetzung nicht oder nur wenig ablaufen. Diese Aufgabe wird durch die Erfindung gelöst.
Gegenstand der Erfindung ist ein Verfahren zum Herstellen von Organopolysiloxanelastomeren durch Vermischen eines Gemisches aus in den endständigen Einheiten je eine Si-gebundene Hydroxylgruppe aufweisendem Diorganopolysiloxan und anorga-nischem Oxyd mit einer Oberfläche von mindestens 50 $m^2/g$ mit Vernetzungsmittel und Kondensationskatalysator und Vernetzenlassen dieser Mischung auf einer abzuformenden Oberfläche, dadurch gekennzeichnet, daß das Gemisch aus in den endständigen Einheiten je eine Si-gebundene Hydroxylgruppe aufweisendem Diorganopolysiloxan und anorganischem Oxyd mit einer Oberfläche von mindestens 50 $m^2/g$ durch Vermischen von in den endständigen Einheiten je eine Si-gebundene Hydroxylgruppe aufweisendem Diorganopolysiloxan mit einer durchschnittlichen Viskosität von 500 bis 6000 mPa.s bei 25°C mit anorganischem Oxyd mit einer Oberfläche von mindestens 50 $m^2/g$ in einer Menge von 2 bis 35 Gewichtsprozent anorganischem Oxyd, bezogen auf das Gesamtgewicht von Diorganopolysiloxan und anorganischem Oxyd, sowie mit Wasser und mit das anorganische Oxyd hydrophobierender Organosiliciumverbindung und Entfernung der niedriger als das Diorganopolysiloxan siedenden Bestandteile aus dem Gemisch und gegebenenfalls erfolgendem weiteren Zusatz von Wasser hergestellt wurde.

Die zur Herstellung der erfindungsgemäß verwendeten Gemische aus in den endständigen Einheiten je eine Si-gebundene Hydroxylgruppe aufweisendem Diorganopolysiloxan und anorganischem Oxyd eingesetzten Diorganopolysiloxane können durch die Formel

$$HOR_2SiO(SiR_2O)_xSiR_2OH$$

wiedergegeben werden. In dieser Formel bedeutet R gleiche oder verschiedene, einwertige, gegebenenfalls substituierte Kohlenwasserstoffreste und x eine Zahl, welche der durchschnittlichen Viskosität von 500 bis 6000 mPa.s bei 25°C entspricht.

Innerhalb bzw. entlang der Siloxankette in der oben angegebenen Formel können, was durch derartige Formeln üblicherweise nicht dargestellt wird, auch andere Siloxaneinheiten als Diorganosiloxaneinheiten ($SiR_2O$) vorliegen. Beispiele für solche anderen, meist jedoch lediglich als mehr oder weniger schwer vermeidbare Verunreinigungen vorliegende Siloxaneinheiten sind solche der Formel $RSiO_{3/2}$, $R_3SiO_{1/2}$ und $SiO_{4/2}$, worin R jeweils die oben dafür angegebene Bedeutung hat. Vorzugsweise beträgt die Menge an solchen anderen Siloxaneinheiten höchstens 5 Molprozent der Siloxaneinheiten in den zur Herstellung der erfindungsgemäß verwendeten Gemische aus in den endständigen Einheiten je eine Si-gebundene Hydroxylgruppe aufweisendem Diorganopolysiloxan und anorganischem Oxyd verwendeten Organopolysiloxanen.

Beispiele für Kohlenwasserstoffreste R sind Alkylreste, wie Methyl-, Ethyl-, Propyl-, Butyl-, Hexyl- und Octylreste; aliphatische Kohlenwasserstoffreste mit Kohlenstoff-Kohlenstoff-Doppelbindungen, wie der Vinyl-, Allyl-, Ethylallyl- und Butadienylrest; Arylreste, wie der Phenylrest; Alkarylreste, wie Tolylreste; und Aralkylreste, wie der Benzylrest.

Beispiele für substituierte Kohlenwasserstoffreste sind insbesondere halogenierte Kohlenwasserstoffreste, wie der 3,3,3-Trifluorpropylrest, Chlorphenyl- und Bromtolylreste, sowie Cyanalkylreste, wie der beta-Cyanethylrest.

Vorzugsweise sind jedoch schon wegen der leichteren Zugänglichkeit mindestens 90 % der Anzahl der Reste R Methylreste.

Die hier in Beschreibung und Patentansprüchen angegebenen Werte für die Größe der Oberfläche des anorganischen Oxyds

sind BET-Werte, also Werte, die durch Stickstoffadsorption gemäß ASTM Special Technical Publication No. 51,1941, Seite 95 ff bestimmt wurden. Vorzugsweise beträgt die Oberfläche des anorganischen Oxyds höchstens 300 $m^2/g$. Eine Oberfläche von 100 bis 160 $m^2/g$ ist besonders bevorzugt.

Als anorganisches Oxyd ist pyrogen erzeugtes Siliciumdioxyd bevorzugt. Weitere Beispiele für im Rahmen der Erfindung verwendbare anorganische Oxyde sind unter Erhaltung der Struktur entwässerte Kieselsäurehydrogele, die auch als "Aerogele" bezeichnet werden, anderes gefälltes Siliciumdioxyd mit einer Oberfläche von mindestens 50 $m^2/g$, pyrogen erzeugtes Aluminiumoxyd und pyrogen erzeugtes Titandioxyd.

Bei der Hydrophobierung des anorganischen Oxyds wird Wasser vorzugsweise in Mengen von 5 bis 12 Gewichtsprozent, bezogen auf das Gewicht des während dieser Hydrophobierung anwesenden Diorganopolysiloxans, verwendet.
Als das anorganische Oxyd hydrophobierende Organosiliciumverbindungen können beliebige Organosiliciumverbindungen eingesetzt werden, mit denen auch bisher in Gegenwart von Wasser und Organopolysiloxan, insbesondere Diorganopolysiloxan, anorganisches Oxyd hydrophobiert werden konnte. Bevorzugt als das anorganische Oxyd hydrophobierende Organosiliciumverbindungen sind solche der Formel

$$(R_3Si)_aZ,$$

worin R die oben dafür angegebene Bedeutung hat, a 1 oder 2 und Z -NX- oder $-NX_2$ ist und X Wasserstoff oder ein Alkylrest mit 1 bis 4 Kohlenstoffatomen ist.

Beispiele für bevorzugte, das anorganische Oxyd hydrophobierende Organosiliciumverbindungen sind Hexamethyldisilazan, das besonders bevorzugt ist, ferner Triorganosilylamine, wie Trimethylsilylisopropylamin, Trimethylsilylethylamin, Dimethylphenylsilyl-n-propylamin und Vinyldimethylsilyl-n-butylamin.

Die anorganisches Oxyd hydrophobierende Organosiliciumverbindung wird vorzugsweise in Mengen von 13 bis 25 Gewichtsprozent, bezogen auf das Gewicht des während dieser Hydrophobierung anwesenden Diorganopolysiloxans, verwendet.

Das Vermischen von Diorganopolysiloxan mit anorganischem Oxyd, Wasser und mit das anorganische Oxyd hydrophobierender Organosiliciumverbindung ist beispielsweise in US 4 101 499, ausgegeben 18. Juli 1978, J. Herzig, Bayer AG, eingehend beschrieben. Dieser Veröffentlichung war jedoch das Ausbleiben vom Absetzen des Füllstoffs nach Lagerung des Gemisches bei verhältnismäßig geringen Füllstoffmengen und verhältnismäßig geringer Viskosität des mit dem Füllstoff vermischten Diorganopolysiloxans nicht zu entnehmen.

Weil das Vermischen von Diorganopolysiloxan mit anorganischem Oxyd, Wasser und mit das anorganische Oxyd hydrophobierender Organosiliciumverbindung beispielsweise aus US 4 101 499 bereits bekannt ist, muß es hier nicht mehr eingehender in allgemeiner Form beschrieben werden. Vorzugsweise werden jedoch im Gegensatz zu der Arbeitsweise gemäß US 4 101 499 die erfindungsgemäß verwendeten Gemische aus in den endständigen Einheiten je eine Si-gebundene Hydroxylgruppe aufweisendem Diorganopolysiloxan und anorganischem Oxyd dadurch hergestellt, daß zunächst nur 35 bis 60 Gewichtsprozent der

insgesamt eingesetzten Menge von in den endständigen Einheiten je eine Si-gebundene Hydroxylgruppe aufweisendem Diorganopolysiloxan mit Wasser, das anorganische Oxyd hydrophobierender Organosiliciumverbindung und dem anorganischen Oxyd vermischt werden, anschließend die niedriger als das Diorganopolysiloxan siedenden Bestandteile aus dem so erhaltenen Gemisch entfernt werden und erst dann dieses Gemisch mit dem Rest der insgesamt eingesetzten Menge von in den endständigen Einheiten je eine Si-gebundene Hydroxylgruppe aufweisendem Diorganopolysiloxan vermischt wird.

Abgesehen von der Temperatur bei der Entfernung der niedriger als das Diorganopolysiloxan siedenden Bestandteile, die vorzugsweise 30° bis 180°C beträgt, wird die Herstellung der erfindungsgemäß verwendeten Gemische aus in den endständigen Einheiten je eine Si-gebundene Hydroxylgruppe aufweisendem Diorganopolysiloxan und anorganischem Oxyd vorzugsweise bei 10°C bis 50°C durchgeführt.

Wird das Gemisch aus in den endständigen Einheiten je eine Si-gebundene Hydroxylgruppe aufweisendem Diorganopolysiloxan und hydrophobiertem anorganischem Oxyd nach der Entfernung der niedriger als das Diorganopolysiloxan siedenden Bestandteile noch einmal mit Wasser vermischt, so vorzugsweise in Mengen von höchstens 1 Gewichtsprozent Wasser, bezogen auf das Gewicht des Gemisches vor diesem Vermischen mit Wasser.

Zusätzlich zu in den endständigen Einheiten je eine Si-gebundene Hydroxylgruppe aufweisendem Diorganopolysiloxan, anorganischem Oxyd, Wasser und das anorganische Oxyd hydrophobierender Organosiliciumverbindung können bei der Herstellung der erfindungsgemäß verwendeten Gemische aus in den endständigen Einheiten je eine Si-gebundene Hydroxylgruppe aufweisendem Diorganopolysiloxan und anorganischem Oxyd gegebenenfalls weitere Stoffe mitverwendet werden. Beispiele für solche weiteren Stoffe sind anorganische Füllstoffe mit einer Oberfläche von weniger als 50 $m^2/g$, wie Quarzmehl und Calciumsilikat, Stoffe zur weiteren Thixotropierung, wie Polyglykole, die verethert oder verestert oder verethert und verestert sein können, einschließlich Organosiloxan-Oxyalkylen-Mischpolymerisaten (US 3 792 147, ausgegeben 12. Februar 1974, E. Wohlfarth et al., Wacker-Chemie GmbH) und hydriertes Rizinusöl, in Diorganopolysiloxanen lösliche Farbstoffe, Duftstoffe und Aromastoffe.

Als Vernetzungsmittel und Kondensationskatalysatoren können bei dem erfindungsgemäßen Verfahren beliebige Vernetzungsmittel und Kondensationskatalysatoren verwendet werden, mit denen auch bisher, insbesondere in der Praxis des Zahnarztes, in den endständigen Einheiten je eine Si-gebundene Hydroxylgruppe aufweisende Diorganopolysiloxane durch Kondensation vernetzt werden konnten. Auch die dabei verwendeten Mengenverhältnisse können die gleichen sein wie bisher.

Bei genauen Abformungen von Zähnen oder Schleimhaut oder von Zähnen und Schleimhaut im Munde von Patienten ist natürlich verhältnismäßig rasche Vernetzung der Massen bei ausreichender Dauer der Verarbeitungszeit (englisch: pot life) erwünscht. Bei der Abformung von feinsten Einzelheiten anderer Oberflächen können selbstverständlich meist längere Vernetzungszeiten in Kauf genommen werden. Wenn aber verhältnismäßig rasche Vernetzung erwünscht ist und dies ist bei der bevorzugten Anwendung des erfindungsgemäßen Verfahrens der Fall, so sind bevorzugt Vernetzungsmittel, die gleichzeitig Kondensationskatalysatoren sind, inbesondere Umsetzungsprodukte von Kieselsäureester mit organischer Zinnverbindung der Formel

$$R^2_2Sn(OCOR^3)_2,$$

worin $R^2$ einen Butyl- oder Octylrest und $R^3$ einen einwertigen Kohlenwasserstoffrest mit 1 bis 15 Kohlenstoffatomen bedeutet, wobei höchstens eine der Valenzen des an die Carboxylgruppe gebundenen Kohlenstoffatoms durch ein anderes Kohlenstoffatom als dasjenige der Carboxylgruppe abgesättigt ist, im Gemisch mit organischer Zinnverbindung der Formel

$$R^2_2\ Sn(OCOR^4)_2,$$

worin $R^2$ die vorstehend dafür angegebene Bedeutung hat und $R^4$ gleiche oder verschiedene aliphatische Kohlenwasserstoffreste mit 3 bis 15 Kohlenstoffatomen bedeutet, wobei mindestens zwei der Valenzen des an die Carboxylgruppe gebundenen Kohlenstoffatoms durch mindestens zwei andere Kohlenstoffatome als dasjenige der Carboxylgruppe abgesättigt sind (US 4 360 654, ausgegeben 23. November 1982, W. Hechtl et al., Wacker-Chemie GmbH).

Weitere Beispiele für Vernetzungsmittel sind bei 20°C und 1000 hPa (abs) flüssige Siliciumverbindungen mit mindestens drei SiOC-gebundenen Alkoxyresten mit 1 bis 4 Kohlenstoffatomen je Molekül, wie Hexaethoxydisiloxan, Tetraethylsilikat, Polyethylsilikat mit einem $SiO_2$-Gehalt von 40 Gewichtsprozent (bekannt unter der Bezeichnung "Ethylsilikat 40") und 1,1,1-Trimethyl-3,3,3-triethoxydisiloxan.

Die oben genannten von Zinn freien Vernetzungsmittel müssen

gemeinsam mit Kondensationskatalysatoren, wie organischen Zinnsalzen, wie Dibutylzinndilaurat, Stannooktoat oder organischen Zinnverbindungen der Formel

$$R^2_2Sn(OCOR^4)_2,$$

worin $R^2$ und $R^4$ jeweils die oben dafür angegebene Bedeutung haben, verwendet werden.

Vernetzungsmittel, die gleichzeitig Kondensationskatalysatoren sind, werden vorzugsweise in Mengen von 2 bis 10 Gewichtsprozent, andere Vernetzungsmittel und andere Kondensationskatalysatoren werden vorzugsweise jeweils in Mengen von 0,1 bis 10 Gewichtsprozent, jeweils bezogen auf das Gewicht von zu vernetzendem Diorganopolysiloxan, eingesetzt.

Vorzugsweise haben die zum Elastomeren vernetzenden Massen, die durch Vermischen der erfindungsgemäß verwendeten Gemische aus in den endständigen Einheiten je eine Si-gebundene Hydroxylgruppe aufweisendem Diorganopolysiloxan und anorganischem Oxyd mit Vernetzungsmittel und Kondensationskatalysator hergestellt wurden, vor dem Beginn einer merklichen Vernetzung eine Viskosität von höchstens 30 000 mPa.s bei 25°C, vorzugsweise höchstens 25 000 mPa.s bei 25°C.

Nach Vermischen der erfindungsgemäß verwendeten Gemische aus in den endständigen Einheiten je eine Si-gebundene Hydroxylgruppe aufweisendem Diorganopolysiloxan und anorganischem Oxyd mit Vernetzungsmittel und Kondensationskatalysator und Auftragen der so erhaltenen, zum Elastomer vernetzenden Masse auf die abzuformende Oberfläche erfolgt dort beim Druck der umgebenden Atmosphäre, meist 1020 hPa (abs.) oder etwa

1020 hPa (abs.), und bei Raumtemperatur bzw. der im Munde eines Patienten herrschenden Temperatur die Vernetzung der Masse. Das so erhaltene Elastomer kann dann von der abzuformenden Oberfläche abgenommen werden.

Das erfindungsgemäße Verfahren eignet sich ausgezeichnet zur Herstellung von Dentalkorrektur-Abdrücken bzw. als Teil der Herstellung von Dentalkorrektur-Abdrücken. Bei der Herstellung von derartigen Abdrücken wird meist in einer 1. Stufe oder Phase mit einer verhältnismäßig hochviskosen bei Raumtemperatur zu einem Elastomeren vernetzenden Masse auf Grundlage von Diorganopolysiloxan ein Vorabdruck gemacht, der noch nicht genau alle Einzelheiten der abzuformenden Oberfläche wiedergibt. In einer 2. Stufe oder Phase wird dann erfindungsgemäß verwendetes Gemisch aus in den endständigen Einheiten je eine Si-gebundene Hydroxylgruppe aufweisendem Diorganopolysiloxan und anorganischem Oxyd mit Vernetzungsmittel und Kondensationskatalysator vermischt, die so erhaltene zum Elastomeren vernetzende Masse in eine bei der Anfertigung von Dentalkorrektur-Abdrücken üblicherweise verwendete Spritze aufgezogen und dann in dünnem Strahl auf den abzuformenden Teil im Patienten-Mund aufgebracht, wobei sie auch in die engen Zwischenräume der Zähne eindringen kann. Noch bevor diese Masse begonnen hat, merklich zu vernetzen, wird der Vorabdruck auf den so überzogenen Teil des Mundes des Patienten aufgeschoben, wodurch die in der 2. Stufe aufgebrachte Masse in noch kleinere, bisher von dieser Masse frei gebliebene Teile der abzuformenden Oberfläche gedrückt wird, bevor sie schließlich vernetzt und sich dabei mit dem Vorabdruck verbindet.

Beispiel

In einen heiz- und kühlbaren Doppelschnecken-Trogkneter mit einem Fassungsvermögen von 3 l werden unter Stickstoff 600 g eines in den endständigen Einheiten je eine Si-gebundene Hydroxylgruppe aufweisenden Dimethylpolysiloxans mit einer Viskosität von 1000 mPa.s bei 25°C, 93,7 g Hexamethyldisilazan und 37,5 g durch Ionenaustauscher gereinigtes Wasser gegeben und 10 Minuten bei etwa 1020 hPa (abs.) und etwa 20°C miteinander vermischt. Dann werden in diesen Troginhalt unter Stickstoff, während er durch Kühlung bei 20°C bis 45°C gehalten wird, innerhalb von 2 Stunden insgesamt 600 g pyrogen erzeugtes Siliciumdioxyd mit einer Oberfläche von etwa 130 $m^2/g$ in 6 gleichen Anteilen eingeknetet, wonach noch etwa 10 Minuten bei höchstens 45°C und etwa 1020 hPa (abs.) weitergeknetet wird. In den so erhaltenen Troginhalt werden unter Stickstoff nochmals 18,7 g Hexamethyldisilazan und 6,2 g durch Ionenaustauscher gereinigtes Wasser gegeben. Dann wird 30 Minuten bei etwa 1020 hPa (abs.) unter Stickstoff geknetet, während der Troginhalt durch Kühlung bei höchstens 45°C gehalten wird. Dann werden unter Stickstoff 133,8 g des in den endständigen Einheiten je eine Si-gebundene Hydroxylgruppe aufweisenden Dimethylpolysiloxans mit einer Viskosität von 1000 mPa.s in 6 gleichen Anteilen innerhalb von 15 Minuten zu dem Troginhalt gegeben, wobei die Temperatur des Troginhalts bei höchstens 40°C gehalten wird und der Druck etwa 1020 hPa (abs.) beträgt. Dann wird zuerst unter Stickstoff 30 Minuten bei höchstens 40°C und etwa 1020 hPa (abs.) und dann ohne Stickstoffzufuhr zur Entfernung von niedriger als das Diorganopolysiloxan siedenden Bestandteilen bei höchstens 50 hPa (abs.) und höchstens 40°C 30 Minuten geknetet. Dann werden wieder unter Stickstoff 30 g Hexamethyldisilazan und 6,2 g durch

Ionenaustauscher gereinigtes Wasser zu dem Troginhalt gegeben, und es wird 30 Minuten bei etwa 1020 hPa (abs.) und höchstens 40°C geknetet. Ohne Stickstoffzufuhr wird dann 30 Minuten bei höchstens 50 hPa (abs.) und höchstens 40°C und danach 2 Stunden bei 150°C geknetet. Dann werden bei etwa 1020 hPa (abs.) und etwa 40°C innerhalb 15 Minuten 466,2 g des in den endständigen Einheiten je eine Si-gebundene Hydroxylgruppe aufweisenden Dimethylpolysiloxans mit einer Viskosität von 1000 mPa.s bei 25°C in 5 gleichen Anteilen eingeknetet und danach wird 10 Minuten weitergeknetet. Schließlich wird die so erhaltene Mischung aus dem Trogkneter durch einen Dreiwalzenstuhl geführt, und dann in ein mit Flügelrührer ausgestattetes Gefäß übergeführt, wo sie zunächst mit 400 g des in den endständigen Einheiten je eine Si-gebundene Hydroxylgruppe aufweisenden Dimethylpolysiloxans mit einer Viskosität von 1000 mPa.s bei 25°C und dann mit 0,5 Gewichtsprozent durch Ionenaustauscher gereinigtes Wasser, bezogen auf das Gesamtgewicht der Mischung vermischt wird. Nach 24 Stunden Lagerung im verschlossenen Gefäß bei Raumtemperatur hat das so erhaltene Gemisch eine Viskosität von 10 400 mPa.s bei 25°C. Nach 6 Monaten Lagerung dieser Flüssigkeit ist keine Absetzung von Füllstoff zu beobachten.

Ein Teil des Gemisches, dessen Herstellung vorstehend beschrieben wurde, wird mit 4 Gewichtsprozent, bezogen auf sein Gewicht, eines Vernetzungsmittels vermischt, dessen Herstellung, wie folgt, erfolgte:

Eine Mischung aus 3 Gewichtsteilen Tetraethylsilikat und 1 Gewichtsteil Di-n-octylzinndiacetat wird 3 Stunden bei einer Badtemperatur von 120°C unter Rückfluß erwärmt. Nachdem das Bad auf 50°C abgekühlt ist, werden die bis zu 50°C bei 22 hPa (abs.) siedenden Bestandteile abdestilliert. Der

Rückstand wird mit 5 Gewichtsprozent, bezogen auf sein Gewicht, Di-n-octylzinn-di-2-ethylhexoat vermischt.

Die Mischung aus in den endständigen Einheiten je eine Si-gebundene Hydroxylgruppe aufweisendem Dimethylpolysiloxan und anorganischem Oxyd wird nach dem Vermischen mit dem Zinn enthaltenden Vernetzungsmittel in eine bei der Anfertigung von Dentalkorrektur-Abdrücken üblicherweise verwendete Spritze aufgezogen und in einem Strahl mit einem Durchmesser von etwa 1,5 mm auf eine Gebiss-Prothese gespritzt, wo sie von den senkrechten Flächen nur unwesentlich abläuft. Noch bevor der Überzug auf dem Gebiss merklich vernetzt, wird ein Vorabdruck, der vorher von der Gebiss-Prothese in bekannter Weise gemacht wurde, auf das so überzogene Gebiß aufgeschoben, wonach innerhalb einiger Minuten der dünne Überzug auf dem Gebiss vernetzt und sich mit dem Vorabdruck zu einem Abdruck des Gebisses verbindet, der die feinsten Einzelheiten der Oberfläche des Gebisses wiedergibt.

Patentansprüche

1. Verfahren zum Herstellen von Organopolysiloxanelastomeren durch Vermischen eines Gemisches aus in den endständigen Einheiten je eine Si-gebundene Hydroxylgruppe aufweisendem Diorganopolysiloxan und anorganischem Oxyd mit einer Oberfläche von mindestens 50 $m^2/g$ mit Vernetzungsmittel und Kondensationskatalysator und Vernetzenlassen dieser Mischung auf einer abzuformenden Oberfläche, dadurch gekennzeichnet, daß das Gemisch aus in den endständigen Einheiten je eine Si-gebundene Hydroxylgruppe aufweisendem Diorganopolysiloxan und anorganischem Oxyd mit einer Oberfläche von mindestens 50 $m^2/g$ durch Vermischen von in den endständigen Einheiten je eine Si-gebundene Hydroxylgruppe aufweisendem Diorganopolysiloxan mit einer durchschnittlichen Viskosität von 500 bis 6000 mPa.s bei 25°C mit anorganischem Oxyd mit einer Oberfläche von mindestens 50 $m^2/g$ in einer Menge von 2 bis 35 Gewichtsprozent anorganischem Oxyd, bezogen auf das Gesamtgewicht von Diorganopolysiloxan und anorganischem Oxyd, sowie mit Wasser und mit das anorganische Oxyd hydrophobierender Organosiliciumverbindung und Entfernung der niedriger als das Diorganopolysiloxan siedenden Bestandteile aus dem Gemisch und gegebenenfalls erfolgendem weiteren Zusatz von Wasser hergestellt wurde.

2. Verfahren nach Anspruch 1, dadurch gekennzeichnet, daß als anorganisches Oxyd mit einer Oberfläche von mindestens 50 $m^2/g$ pyrogen erzeugtes Siliciumdioxyd mit einer Oberfläche von 100 bis 160 $m^2/g$ verwendet wird.

3. Verfahren nach Anspruch 1 oder 2, dadurch gekennzeichnet, daß als das anorganische Oxyd hydrophobierender Organosiliciumverbindung Hexamethyldisilazan verwendet wird.

4. Verfahren nach mindestens einem der Ansprüche 1 bis 3, dadurch gekennzeichnet, daß beim Vermischen von in den endständigen Einheiten je eine Si-gebundene Hydroxylgruppe aufweisendem Diorganopolysiloxan mit anorganischem Oxyd zunächst nur 35 bis 60 Gewichtsprozent der insgesamt eingesetzten Menge von in den endständigen Einheiten je eine Si-gebundene Hydroxylgruppe aufweisendem Diorganopolysiloxan mit Wasser, das anorganische Oxyd hydrophobierender Organosiliciumverbindung und dem anorganischen Oxyd vermischt werden, anschließend die niedriger als das Diorganopolysiloxan siedenden Bestandteile aus dem so erhaltenen Gemisch entfernt werden und erst dann dieses Gemisch mit dem Rest der insgesamt eingesetzten Menge von in den endständigen Einheiten je eine Si-gebundene Hydroxylgruppe aufweisendem Diorganopolysiloxan vermischt wurde.

5. Verfahren nach mindestens einem der Ansprüche 1 bis 4, dadurch gekennzeichnet, daß als Vernetzungsmittel und gleichzeitig Kondensationskatalysatoren Umsetzungsprodukte von Kieselsäureester mit organischer Zinnverbindung der Formel

$$R^2_2Sn(OCOR^3)_2,$$

worin $R^2$ einen Butyl- oder Octylrest und $R^3$ einen einwertigen Kohlenwasserstoffrest mit 1 bis 15 Kohlenstoffatomen

bedeutet, wobei höchstens eine der Valenzen des an die Carboxylgruppe gebundenen Kohlenstoffatoms durch ein anderes Kohlenstoffatom als dasjenige der Carboxylgruppe abgesättigt ist, im Gemisch mit organischer Zinnverbindung der Formel

$$R^2_2Sn(OCOR^4)_2,$$

worin $R^2$ die vorstehend dafür angegebene Bedeutung hat und $R^4$ gleiche oder verschiedene aliphatische Kohlenwasserstoffreste mit 3 bis 15 Kohlenstoffatomen bedeutet, wobei mindestens zwei der Valenzen des an die Carboxylgruppe gebundenen Kohlenstoffatoms durch mindestens zwei andere Kohlenstoffatome als dasjenige der Carboxylgruppe abgesättigt sind, verwendet werden.

6. Anwendung von Verfahren nach mindestens einem der Ansprüche 1 bis 5 bei der Herstellung von Dentalkorrektur-Abdrücken bzw. als Teil der Herstellung von Dentalkorrektur-Abdrücken.